# EUROPEAN PATENT APPLICATION

(11) **EP 4 170 033 A1**
(43) Date of publication of application: **26.04.2023**
(21) Application number: 21826793.8
(22) Date of filing: 16.06.2021
(51) Int. Cl.: C12N 15/53, C12N 1/21, C12P 13/12, C12P 13/20

(54) **TRANSFORMANT OF GENUS HYDROGENOPHILUS BACTERIUM CAPABLE OF PRODUCING ASPARTIC ACID AND METHIONINE**

(30) Priority: 17.06.2020 JP 2020104487; 07.10.2020 JP 2020169550
(71) Applicant: SUMITOMO CHEMICAL COMPANY, LIMITED, Tokyo 103-6020 (JP); Utilization of Carbon Dioxide Institute Co.,Ltd., Tokyo 135-0064 (JP)
(72) Inventor: KASAI, Nobuyuki, Osaka-shi, Osaka 554-8558 (JP); SUEOKA, Hideaki, Osaka-shi, Osaka 554-8558 (JP); YUKAWA, Hideaki, Tokyo 135-0064 (JP); OHTANI, Naoto, Tokyo 135-0064 (JP)
(74) Representative: J A Kemp LLP
(86) International application number: PCT/JP2021/022884
(87) International publication number: WO 2021/256511

(57) **Abstract**

The present invention provides a tranformant which is produced by introducing an aspartic acid dehydrogenase gene into a bacterium belonging to the genus Hydrogenophilus.

## Description

### TECHNICAL FIELD

The present application claims the priority and benefits under the Paris Convention based on Japanese Patent Application No. 2020-104487 (filed on June 17, 2020) and Japanese Patent Application No. 2020-169550 (filed on October 7, 2020), the entire contents of which are incorporated herein by reference.

The present invention relates to a Hydrogenophilus bacterium transformant having an ability to produce aspartic acid and/or methionine and a method for producing aspartic acid and/or methionine using the same.

### BACKGROUND ART

The Paris Agreement, which was adopted in 2015, provides that global emissions of greenhouse gases should be promptly reduced. Under the Agreement, Japan set the goal of reducing her emission of greenhouse gases such as carbon dioxide and methane by 26% compared with year 2013 levels, by the year 2030. Worldwide, the majority of the production of chemicals depends on petroleum resources, exacerbating the problem of increased greenhouse gas emissions. Accordingly, departure from petroleum dependency is a desirable strategy for the production of chemicals, and research and development of biorefineries that produce green chemicals from biomass is being earnestly carried out in various countries. However, the saccharification of biomass for use as raw materials of microbial fermentation requires complicated processes, besides being costly. As part of research geared towards departure from petroleum dependency, gases such as carbon dioxide, methane and carbon monoxide have attracted attention as more sustainable carbon sources, and techniques for producing valuable chemicals and biofuels using microorganisms that utilize these gases are highly focused. In particular, since carbon fixation is a significant contributor to global warming, efficient utilization of carbon dioxide by fixation of carbon dioxide is highly anticipated.

Aspartic acid is an amino acid which is useful as a raw material for aspartame sweeteners and biodegradable polymers, and methionine is an amino acid which is useful for various chemical products such as pharmaceuticals and feed additives, and as a raw material for the chemical products. In the current industrial processes using petroleum raw materials, aspartic acid is mainly produced from fumaric acid using aspartase, and this fumaric acid is produced by chemical synthesis as a fractionated product of petroleum. Methionine is produced by refining Liquefied Petroleum Gas (LPG) while producing carbon dioxide.

On the other hand, a process for producing aspartic acid and methionine with a genetically modified microorganism has been proposed. For example, a process for producing aspartic acid with a transformant in which an exogenous aspartate dehydrogenase gene has been introduced into an Enterobacteriaceae bacterium such as Escherichia coli or yeast has been proposed (Patent Documents 1 and 2). However, these methods require high-cost saccharides for the growth and culture of microorganisms unlike the present invention in which carbon dioxide is used for the growth and culture of microorganisms. Further, these methods do not contribute sufficiently to global warming.

### CITATION LIST

### PATENT DOCUMENT

Patent Document 1: Japanese Patent No. 5846122
Patent Document 2: WO 2017/083683

### SUMMARY OF THE INVENTION

### PROBLEMS TO BE SOLVED BY INVENTION

The problem to be solved by the present invention is to provide a transformant of a Hydrogenophilus bacterium that is capable of producing aspartic acid and/or methionine utilizing carbon dioxide as a sole carbon source, and a method for producing aspartic acid and/or methionine using the transformant.

### MEANS TO SOLVE PROBLEMS

In order to address the problem including the high cost for raw materials such as saccharides and the above-mentioned global warming, the present inventors have been focused on a Hydrogenophilus bacterium as a microorganism which is able to fix carbon dioxide on an industrial scale. This bacterium carries an enzyme that catalyzes the reaction of producing aspartic acid from oxaloacetic acid, but needs glutamic acid as a substrate. Hence, in order to confer an ability to produce aspartic acid and methionine as a metabolic product thereof on an industrial scale, it is necessary to introduce a gene for an enzyme which catalyzes the reaction of producing aspartic acid from an amino group derived from an inorganic salt. Meanwhile, the present inventors have found that a functional protein is not often expressed or enough expressed even though a heterologous gene is introduced to a Hydrogenophilus bacterium using a vector that functions within a Hydrogenophilus bacterium. It has also been clarified that while genes can be expressed within bacteria other than a Hydrogenophilus bacterium, the genes are often not expressed or not enough expressed within the Hydrogenophilus bacterium.

Under this situation, when the present inventors have studied DNAs for genes predicted as an aspartate dehydrogenase gene in various types of microorganism genomes (hereinafter referred to as aspartate dehydrogenase gene) and construction of vectors for introducing the DNAs into a Hydrogenophilus bacterium, they have found out that introducing DNAs of aspartate dehydrogenase genes derived from the below-mentioned specific microorganisms into a Hydrogenophilus bacterium can efficiently produce aspartic acid and/or methionine. Further, they have found that transformants produced by introducing the DNAs can efficiently produce aspartic acid and/or methionine using carbon dioxide as a sole carbon source.

The present invention has been completed based on the above-mentioned findings, and provides as follows.
[1] A transformant produced by introducing an aspartate dehydrogenase gene into a Hydrogenophilus bacterium.
[2] The transformant according to [1], wherein the aspartate dehydrogenase gene is a DNA selected from any of the followings (1) to (6):
   (1) DNA, which consists of a base sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, or SEQ ID NO: 12;
   (2) DNA, which consists of a base sequence having 90% or more identity to any one of SEQ ID NOs: 1 to 12, and encodes a polypeptide having aspartate dehydrogenase activity;
   (3) DNA, which hybridizes with a DNA consisting of a base sequence complementary to any one of SEQ ID NOs: 1 to 12 under a stringent condition, and encodes a polypeptide having aspartate dehydrogenase activity;
   (4) DNA, which encodes a polypeptide consisting of an amino acid sequence of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, or SEQ ID NO 27;
   (5) DNA, which encodes a polypeptide consisting of an amino acid sequence having 90% or more identity to any one of SEQ ID NOs: 16 to 27, and the polypeptide having aspartate dehydrogenase activity; and
   (6) DNA, which encodes a polypeptide consisting of an amino acid sequence having a deletion, substitution, or addition of one or more amino acids in any one of SEQ ID NOs: 16 to 27, and the polypeptide having aspartate dehydrogenase activity.
[3] The transformant according to [1] or [2], wherein the aspartate dehydrogenase gene is a DNA selected from any of the followings (1) to (6):
   (1) DNA, which consists of a base sequence of SEQ ID NO: 3, SEQ ID NO: 8, SEQ ID NO: 9, or SEQ ID NO: 11;
   (2) DNA, which consists of a base sequence having 90% or more identity to SEQ ID NO: 3, SEQ ID NO: 8, SEQ ID NO: 9, or SEQ ID NO: 11, and encodes a polypeptide having aspartate dehydrogenase activity;
   (3) DNA, which hybridizes with a DNA consisting of a base sequence complementary to SEQ ID NO: 3, SEQ ID NO: 8, SEQ ID NO: 9, or SEQ ID NO: 11 under a stringent condition, and encodes a polypeptide having aspartate dehydrogenase activity;
   (4) DNA, which encodes a polypeptide consisting of an amino acid sequence of SEQ ID NO: 18, SEQ ID NO: 23, SEQ ID NO: 24, or SEQ ID NO: 26;
   (5) DNA, which encodes a polypeptide consisting of an amino acid sequence having 90% or more identity to SEQ ID NO: 18, SEQ ID NO: 23, SEQ ID NO: 24, or SEQ ID NO: 26, and the polypeptide having aspartate dehydrogenase activity; and
   (6) DNA, which encodes a polypeptide consisting of an amino acid sequence having a deletion, substitution, or addition of one or more amino acids in an amino acid sequence of SEQ ID NO: 18, SEQ ID NO: 23, SEQ ID NO: 24, or SEQ ID NO: 26, and the polypeptide having aspartate dehydrogenase activity.
[4] The transformant according to any one of [1] to [3], wherein the Hydrogenophilus bacterium is Hydrogenophilus thermoluteolus.
[5] The transformant according to [4], wherein the Hydrogenophilus thermoluteolus is Hydrogenophilus thermoluteolus TH-1C strain.
[6] A method for producing aspartic acid, which comprises a step of culturing the transformant according to any one of [1] to [5] using carbon dioxide as a substantially sole carbon source.
[7] A method for producing methionine, which comprises a step of culturing the transformant according to any one of [1] to [5] using carbon dioxide as a substantially sole carbon source.

### EFFECT OF INVENTION

The present invention can produce aspartic acid and/or methionine as a metabolic product thereof by introducing an aspartate dehydrogenase gene into a Hydrogenophilus bacterium to work in the bacterium.

### MODE FOR CARRYING OUT THE INVENTION

### (1) Transformant capable of producing aspartic acid and/or methionine

First, the present invention provides a transformant produced by introducing an aspartate dehydrogenase gene into a Hydrogenophilus bacterium. The transformant of the present invention has aspartate dehydrogenase activity, which allows producing aspartic acid and/or methionine as a metabolic product thereof utilizing carbon dioxide as a sole carbon source. That is, the transformant of the present invention is characterized in that it can directly fix carbon dioxide to produce aspartic acid and/or methionine as a raw material of various chemical products on an industrial scale.

The aspartate dehydrogenase (hereinafter also referred to as "AspDH") gene which is introduced in the present invention may be derived from any resources as long as it allows exerting aspartate dehydrogenase activity in a Hydrogenophilus bacterium to be introduced. The aspartate dehydrogenase gene used in the present invention may be a DNA of the aspartate dehydrogenase gene isolated from naturally occurring bacteria or a DNA artificially synthesized using any method known to a person skilled in the art. Further, the aspartate dehydrogenase gene used in the present invention may be a DNA of the aspartate dehydrogenase gene derived from various known organisms or a DNA in which these DNAs are genetically modified. The aspartate dehydrogenase gene used in the present invention is not necessarily identified as an aspartate dehydrogenase gene, and can be a DNA encoding an amino acid sequence of a polypeptide having aspartate dehydrogenase activity. The aspartate dehydrogenase activity used herein means enzymatic activity which produces aspartic acid from oxaloacetic acid and ammonia (NH₃).

The aspartate dehydrogenase gene introduced in the present invention may be, for example, a DNA consisting of the base sequence of the aspartate dehydrogenase gene derived from Candidatus Hydrothermae bacterium (SEQ ID NO: 1), the base sequence of the aspartate dehydrogenase gene derived from Glaciecola sp. 33A (SEQ ID NO: 2), the base sequence of the aspartate dehydrogenase gene derived from Mesorhizobium sp. M7D.F.Ca.US.005.01.1.1 (SEQ ID NO: 3), the base sequence of the aspartate dehydrogenase gene derived from Rhodospirillaceae bacterium (SEQ ID NO: 4), the base sequence of the aspartate dehydrogenase gene derived from Bacillus kochii (SEQ ID NO: 5), the base sequence of the aspartate dehydrogenase gene derived from Novosphingobium rosa (SEQ ID NO: 6), the base sequence of the aspartate dehydrogenase gene derived from Archaeoglobus fulgidus (SEQ ID NO: 7), the base sequence of the aspartate dehydrogenase gene derived from Arthrobacter sp. 161MFSha2.1 (SEQ ID NO: 8), the base sequence of the aspartate dehydrogenase gene derived from Oceanotoga teriensis (SEQ ID NO: 9), the base sequence of the aspartate dehydrogenase gene derived from Marinitoga sp. 1155 (SEQ ID NO: 10), the base sequence of the aspartate dehydrogenase gene derived from Thermotogales bacterium 46_20 (SEQ ID NO: 11), or the base sequence of the aspartate dehydrogenase gene derived from Thermoga maritima (SEQ ID NO: 12).

The aspartate dehydrogenase gene used in the present invention may be a DNA, which consists of a base sequence having 20% or more, 30% or more, 40% or more, 50% or more, 60% or more, 70% or more, 80% or more, 90% or more, 95% or more, 96% or more, 97% or more, 98% or more, or 99% or more identity to any of SEQ ID NOs: 1 to 12, and encodes a polypeptide having aspartate dehydrogenase activity. The aspartate dehydrogenase gene used in the present invention is preferably a DNA, which consists of a base sequence having 90% or more identity to SEQ ID NO: 3, 8, 9, or 11, and encodes a polypeptide having aspartate dehydrogenase activity. In the present invention, the identity of a base sequence can be determined using any method known to a person skilled in the art and can be calculated using, for example, GENETYX ver.17 (manufactured by Genetics Corporation).

The aspartate dehydrogenase gene used in the present invention may be a DNA, which hybridizes with a DNA consisting of a base sequence complementary to any of SEQ ID NOs: 1-12 under a stringent condition and encodes a polypeptide having aspartate dehydrogenase activity. The "stringent condition" as used herein means, for example, a condition of hybridizing with a hybridization solution of 6 x SSC salt concentration solution at temperatures from 50 to 60°C for 16 hours and then washing the DNA in 0.1 x SSC salt concentration solution. The "hybridizing under a stringent condition" as used herein includes specific hybridization, for example, forming a hybrid with 60% or more, 70% or more, 80% or more, 90% or more, 95% or more, 98% or more, or 99% homology, and does not include non-specific hybridization, for example, a hybridization not forming any hybrid with the homology having below the above homology level.

The aspartate dehydrogenase gene introduced in the present invention is preferably the aspartate dehydrogenase gene derived from Candidatus Hydrothermae bacterium, Glaciecola sp. 33A, Mesorhizobium sp. M7D.F.Ca.US.005.01.1.1, Rhodospirillaceae bacterium, Bacillus kochii, Novosphingobium rosa, Archaeoglobus fulgidus, Arthrobacter sp. 161MFSha2.1, Oceanotoga teriensis, Marinitoga sp. 1155, Thermotogales bacterium 46_20, or Thermoga maritima, and more preferably the aspartate dehydrogenase gene derived from Mesorhizobium sp. M7D.F.Ca.US.005.01.1.1, Arthrobacter sp. 161MFSha2.1, Oceanotoga teriensis, or Thermotogales bacterium 46_20 in terms that they can effectively produce aspartic acid and/or methionine. It was predicted that the base sequences of the aspartate dehydrogenase genes derived from the above-recited bacteria except for Thermoga maritima and Archaeoglobus fulgidus (SEQ ID NOs: 1 to 6 and 8 to 11) correspond to a DNA encoding an aspartate dehydrogenase gene based on the genome sequences of the above-recited bacteria, but it could be not easily predictable that the present transformants have aspartate dehydrogenase activity because the amino acid sequences encoded by the above-recited base sequences (SEQ ID NOs: 16 to 21 and 23 to 26) have a significantly low homology of up to 50% with each amino acid sequence of aspartate dehydrogenases derived from other microorganisms which have been confirmed to have the aspartate dehydrogenase activity.

The aspartate dehydrogenase gene used in the present invention may be a DNA, which encodes the polypeptide consisting of the amino acid sequence of the aspartate dehydrogenase gene derived from Candidatus Hydrothermae bacterium (SEQ ID NO: 16), the amino acid sequence of the aspartate dehydrogenase gene derived from Glaciecola sp. 33A (SEQ ID NO: 17), the amino acid sequence of the aspartate dehydrogenase gene derived from Mesorhizobium sp. M7D.F.Ca.US.005.01.1.1 (SEQ ID NO: 18), the amino acid sequence of the aspartate dehydrogenase gene derived from Rhodospirillaceae bacterium (SEQ ID NO: 19), the amino acid sequence of the aspartate dehydrogenase gene derived from Bacillus kochii (SEQ ID NO: 20), the amino acid sequence of the aspartate dehydrogenase gene derived from Novosphingobium rosa (SEQ ID NO: 21), the amino acid sequence of the aspartate dehydrogenase gene derived from Archaeoglobus fulgidus (SEQ ID NO: 22), the amino acid sequence of the aspartate dehydrogenase gene derived from Arthrobacter sp. 161MFSha2.1 (SEQ ID NO: 23), the amino acid sequence of the aspartate dehydrogenase gene derived from Oceanotoga teriensis (SEQ ID NO: 24), the amino acid sequence of the aspartate dehydrogenase gene derived from Marinitoga sp. 1155 (SEQ ID NO: 25), the amino acid sequence of the aspartate dehydrogenase gene derived from Thermotogales bacterium 46_20 (SEQ ID NO: 26), or the amino acid sequence of the aspartate dehydrogenase gene derived from Thermoga maritima (SEQ ID NO: 27).

In addition, the aspartate dehydrogenase gene used in the present invention may be a DNA, which encodes a polypeptide consisting of an amino acid sequence having 20% or more, 30% or more, 40% or more, 50% or more, 60% or more, 70% or more, 80% or more, 90% or more, 95% or more, 96% or more, 97% or more, 98% or more, or 99% or more identity to any of SEQ ID NOs: 16 to 27, and the polypeptide having aspartate dehydrogenase activity. The aspartate dehydrogenase gene used in the present invention is preferably a DNA, which encodes a polypeptide consisting of an amino acid sequence having 90% or more identity to SEQ ID NO: 18, 23, 24, or 26, and the polypeptide having aspartate dehydrogenase activity. In the present invention, the identity of an amino acid sequence can be determined using any method known to a person skilled in the art and can be calculated using, for example, GENETYX ver.17 (manufactured by Genetics Corporation).

Further, the aspartate dehydrogenase gene used in the present invention may be a DNA, which encodes a polypeptide consisting of an amino acid sequence having a deletion, substitution, or addition of one or more amino acids in any of the amino acid sequences of SEQ ID NOs: 16 to 27, and the polypeptide having aspartate dehydrogenase activity. The one or more amino acids used herein include, for example, one to five, one to four, one to three, one to two, or one amino acids.

The aspartate dehydrogenase gene introduced in the present invention is preferably a DNA, which encodes the polypeptide consisting of the amino acid sequence of the aspartate dehydrogenase derived from Candidatus Hydrothermae bacterium, Glaciecola sp. 33A, Mesorhizobium sp. M7D.F.Ca.US.005.01.1.1, Rhodospirillaceae bacterium, Bacillus kochii, Novosphingobium rosa, Archaeoglobus fulgidus, Arthrobacter sp. 161MFSha2.1, Oceanotoga teriensis, Marinitoga sp. 1155, Thermotogales bacterium 46_20, or Thermoga maritima (SEQ ID NOs: 16 to 27), more preferably a DNA, which encodes the polypeptide consisting of the amino acid sequence of the aspartate dehydrogenase gene derived from Mesorhizobium sp. M7D.F.Ca.US.005.01.1.1, Arthrobacter sp. 161MFSha2.1, Oceanotoga teriensis, or Thermotogales bacterium 46_20 in terms that they can effectively produce aspartic acid and/or methionine.

The aspartate dehydrogenase activity as used herein can be measured and determined with any method commonly used in the art. The aspartate dehydrogenase activity can be confirmed, for example, by reacting the produced polypeptide with oxaloacetic acid in the presence of NADH and detecting the absorbance at 340 nm. The aspartate dehydrogenase produces aspartic acid from oxaloacetic acid. Since the aspartate dehydrogenase consumes NADH in producing aspartic acid from oxaloacetic acid, a decrease in the amount of NADH can be detected using a decrease in the absorbance at 340 nm as an indicator. It can be determined that a test polypeptide has aspartate dehydrogenase activity if it reduces the absorbance at 340 nm even by any amount.

### (2) Method for producing transformants

Next, a method for introducing an aspartate dehydrogenase gene into a Hydrogenophilus bacterium to obtain a transformant is described. First, the Hydrogenophilus bacterium to which the aspartate dehydrogenase gene is introduced as used herein is not particularly limited, and may be isolated from the natural environment or be genetically modified to highly express the introduced aspartate dehydrogenase gene. The modification can be performed, for example, by removing any endogenous plasmid from the Hydrogenophilus bacterium. A method for removing (curing) an endogenous plasmid which can be used in the present invention may be any method known in the art including, but not limited to, for example, a method using a chemical substance (for example, a method using novobiocin, SDS, acryflavin, ethidium bromide, etc.), a method using plasmid incompatibility (for example, a method of destabilizing an endogenous plasmid by introducing a plasmid carrying the same replication origin as the endogenous plasmid, and a method of destabilizing an endogenous plasmid by destroying factors related to the plasmid partition system, etc.).

The Hydrogenophilus bacterium which can be used in the present invention includes, for example, Hydrogenophilus thermoluteolus, Hydrogenophilus halorhabdus, Hydrogenophilus denitrificans, Hydrogenophilus hirschii, Hydrogenophilus islandicus, and Hydrogenophilus sp. Mar3, and is preferably Hydrogenophilus thermoluteolus having a high growth rate and carbon fixation ability as a carbon dioxide fixing microorganism, in particular Hydrogenophilus thermoluteolus strain TH-1 (NBRC 14978). Hydrogenophilus thermoluteolus strain TH-1 exhibits the highest rapid growth among carbon dioxide fixing microorganisms (Agricultural and Biological Chemistry, 41, 685-690 (1977)). Hydrogenophilus thermoluteolus strain TH-1 (NBRC 14978) is internationally deposited under the Budapest Treaty. Further, the most preferable bacterium strain used in the present invention is Hydrogenophilus thermoluteolus strain TH-1C, which can be prepared according to the Examples of the present specification.

The introduction of the aspartate dehydrogenase gene into the Hydrogenophilus bacterium to prepare the present transformant can be performed with any conventional method used in the art to introduce an exogenous gene into a bacterium. The introduction of the aspartate dehydrogenase gene into the Hydrogenophilus bacterium as used herein may be performed by directly introducing an aspartate dehydrogenase gene into a Hydrogenophilus bacterium, or by inserting an aspartate dehydrogenase gene to a vector for transformation (for example, plasmid vector, virus vector, cosmid, fosmid, BAC, and YAC, etc.) and then introducing the vector into a Hydrogenophilus bacterium. The vector for the introduction used in the present invention may be any vector commonly used in the art as long as the vector has an ability to autonomously replicate inside a Hydrogenophilus bacterium, and include, for example, a broad-host-range vector including pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), or a genetically modified vector thereof (e.g. pCAMO-4). The vector for the introduction used in the present invention is preferably pCAMO-4, which can be prepared according to the following examples by a person skilled in the art. A promoter contained in the vector used in the present invention includes, for example, tac promoter, lac promoter, trc promoter, and each promotor of OXB1 and OXB11 to OXB20 manufactured by Oxford Genetics Ltd, and a preferred terminator includes T1T2 terminator of Escherichia coli rRNA operon rrnB, t0 transcription terminator of bacteriophage λ, and T7 terminator. A method for introducing (transforming) the aspartate dehydrogenase gene into the Hydrogenophilus bacterium as used herein may be any conventional method used in the art to introduce the vector so that it can autonomously replicate in the Hydrogenophilus bacterium, and includes calcium chloride method, calcium phosphate method, DEAE dextran-mediated transfection method, and electric pulse method (electroporation method) and the others.

### Method for producing aspartic acid and/or methionine

The present invention provides, in a further aspect, a method for producing aspartic acid and/or methionine using the transformant described herein. The method of the present invention may comprise a step of culturing the transformant described herein using carbon dioxide as a substantially sole carbon source. The culturing step in the method of the present invention may comprise culturing the transformant using an inorganic medium or an organic medium while supplying a mixed gas containing hydrogen, oxygen, and carbon dioxide. The gas to be supplied is preferably a mixed gas composed of hydrogen, oxygen, and carbon dioxide, but other gasses may be mixed in as long as aspartic acid and/or methionine can be efficiently produced.

The transformant Hydrogenophilus bacterium used in the method of the present invention can effectively fix carbon dioxide, in particular, by producing the above compounds using carbon dioxide as a substantially sole carbon source (in particular, using only carbon dioxide) because it can grow using hydrogen as an energy source and carbon dioxide as a sole carbon source. Hence, the method of the present invention preferably comprises using an inorganic medium containing no carbon source such as organic substance and carbonate, particularly culturing with only carbon dioxide (in particular, using only carbon dioxide) as a sole carbon source. The "using carbon dioxide as a substantially sole carbon source" as used herein may include the case where an unavoidable amount of other carbon sources is mixed. The method of the present invention can comprise using a medium containing organic substances such as saccharides, organic acids and amino acids and carbonates without supplying carbon dioxide. A pH of the medium used in the method of the present invention is preferably 6.2 to 8, more preferably 6.4 to 7.4, even more preferably 6.6 to 7. Within this range, the growth of bacteria and the solubility of the mixed gas in the medium are high, and thus aspartic acid and/or methionine can be produced with high efficiency. In the method of the present invention, when batch culture is used, the mixed gas can be enclosed in a closed culture container for static culture or shaking culture, and when continuous culture is used, the mixed gas is continuously supplied to a closed culture container for shaking culture or the transformant may be cultured using a closed culture container while introducing the mixed gas into the medium by bubbling. The method of the present invention preferably comprises shaking culture because the dissolution of the mixed gas in the medium is improved. The volume ratio of hydrogen, oxygen, and carbon dioxide (hydrogen : oxygen : carbon dioxide) in the supply gas used in the method of the present invention is preferably 1.75 to 7.5 : 1 : 0.25 to 3, more preferably 5 to 7.5 : 1 : 1. 2, even more preferably 6.25 to 7.5 : 1 : 1.5. Within this range, the growth of the bacterium is good, and thus the target compounds can be efficiently produced. The supply rate of the mixed gas or raw material gas that can be used in the method of the present invention is 10.5 to 60 L/hour, preferably 10.5 to 40 L/hour, more preferably 10.5 to 21 L/hour per 1L of the medium. Within this range, the transformant the growth of the transformant is good, and thus the target compounds can be efficiently produced and waste of the mixed gas can be suppressed. A culture temperature used in the method of the present invention is preferably 35 to 55°C, more preferably 37 to 52°C, further more preferably 50 to 52°C. Within this range, the growth of the transformant is good, and thus aspartic acid and/or methionine can be efficiently produced.

The method of the present invention can produce aspartic acid and/or methionine in the culture medium by culturing as described above. Aspartic acid and/or methionine can be recovered by recovering the culture medium, but aspartic acid and/or methionine can be also separated from the culture medium by any known methods. Such known methods include precipitation method, distillation method, chromatography, and electro dialysis method, etc.

### EXAMPLE

Next, the present invention is described while referring to the examples. The scope of the present invention is not limited by the following examples.

### Construction of plasmid vector (pCAMO-4)

The method for constructing the plasmid vector used to introduce AspDH gene (pCAMO-4) is described below.

### (1) Preparation of DNA fragment of tac promoter

The following pair of primers was synthesized and used to amplify a DNA fragment of tac promoter using the New England Biolabs plasmid pMAL-c5X as a template. PCR was performed according to a conventional method using "DNA thermal cycler" manufactured by Life Technologies Inc. and KOD FX Neo (manufactured by Toyobo Co., Ltd.) as a reaction reagent.

### Primers for the amplification of Tac promoter

(a-1) 5'-TTTTATAACCCGGGCCATCGACTGCACGGTGCACC-3' (SEQ ID NO: 31)
(b-1) 5'-TGCTAGCACTGTTTCCTGTGTGAAATTGTTATCCG-3' (SEQ ID NO: 32)
SmaI restriction site has been added to primer (a-1) as shown by the underline.

2 µL of the prepared reaction solution was electrophoresed on a 1% agarose gel, and a DNA fragment of about 0.3 kbp corresponding to the tac promoter was detected.

### (2) Preparation of DNA fragment in multicloning region

In order to prepare a DNA fragment of the multicloning region, the following pair of primers was synthesized and used for PCR to prepare the former and last parts of the DNA fragment. PCR was performed according to a conventional method using "DNA thermal cycler" manufactured by Life Technologies Inc. and KOD FX Neo (manufactured by Toyobo Co., Ltd.) as a reaction reagent. Actually, this is the reaction in which the 3'-end of each pair of primers are annealed and elongated without containing the template DNA to form a double-stranded DNA.

### Primers for the preparation of the former part in the multicloning region

(a-2) 5'-GGAAACAGTGCTAGCAGATCTGGAGGAGAAACGCATAT-3' (SEQ ID NO: 33) 
(b-2) 5′-CAGTGCGGCCGCAAGCTTGTCGACGGAGCTCGAATTCGGATCCGATATCA

GCATATGCGTTTCTCCTCCAGA-3′ (SEQ ID NO: 34)
 The base sequences of 3'-ends of the primers (a-2) and (b-2) are complementary to each other.
Primers for the preparation of the last part in the multicloning region 
(a-3) 5′-ACAAGCTTGCGGCCGCACTGCAGCACCATCACCACCATCATTGATAAGAT

CCGGCTGCTAACAAAGCCCGAAAGGAAGCT-3′ (SEQ ID NO: 35)
 
(b-3) 5′-TATTTGAATCGAGTTATTGCTCAGCGGTGGCAGCAGCCAACTCAGCTTCC

TTTCGGGCTTTGT-3′ (SEQ ID NO: 36)
The base sequences of 3'-ends of the primers (a-3) and (b-3) are complementary to each other.

20 µL of the above-prepared reaction solution was electrophoresed on a 1% agarose gel, and each DNA fragment of about 0.1 kbp each corresponding to the former and last parts in the multicloning region was detected. Each DNA fragment was cut out from the agarose gel, and the gel was frozen and thawed to recover a DNA. Overlap extension PCR was performed using the recovered DNA fragments corresponding to the former and last parts in the multicloning region as a template. The base sequences at the 5'-ends of the primers (b-2) and (a-3) used to amplify this template DNA fragment are complementary to each other. In the overlap extension PCR, the combination of the primers (a-2) and (b-3) described above was used to prepare the DNA for the multicloning region. PCR was performed according to a conventional method using "DNA thermal cycler" manufactured by Life Technologies Inc. and KOD FX Neo (manufactured by Toyobo Co., Ltd.) as a reaction reagent.

2 uL of the prepared reaction solution was electrophoresed on a 1% agarose gel, and a DNA fragment of about 0.2 kbp corresponding to the tac promoter was detected.

### (3) Preparation of DNA fragment in which rrnB terminator and replication origin region of pUC19 have been linked

The following pair of primers was synthesized and used to amplify the DNA fragment of rrnB terminator using the New England Biolabs plasmid pMAL-c5X as a template.

### Primers for the amplification of rrnB terminator

(a-4) 5'-TAACTCGATTCAAATAAAACGAAAGGCTCAGTCGA-3' (SEQ ID NO: 37)
(b-4) 5'-CCTAGATCCGCGGAGTTTGTAGAAACGCAAAAAGG-3' (SEQ ID NO: 38)

The following pair of primers was synthesized and used to amplify the DNA fragment of the replication origin region using plasmid pUC19 as a template.

### Primers for the amplification of the DNA replication origin region of pUC19

(a-5) 5'-ACAAACTCCGCGGATCTAGGTGAAGATCCTTTTTG-3' (SEQ ID NO: 39)
(b-5) 5'-CGTCCGCGGCCAGCAAAAGGCCAGGAACCGTAAAA-3' (SEQ ID NO: 40)

PCR was performed according to a conventional method using "DNA thermal cycler" manufactured by Life Technologies Inc. and KOD FX Neo (manufactured by Toyobo Co., Ltd.) as a reaction reagent. Each 20 µL of the prepared reaction solution was electrophoresed on a 1% agarose gel, and each DNA fragment of about 0.3 kbp corresponding to rrnB terminator and about 0.8 kbp corresponding to the DNA replication origin region of pUC19 was detected. Each DNA fragment was cut out from the agarose gel, and the gel was frozen and thawed to recover a DNA. Overlap extension PCR was performed using the recovered DNA fragments corresponding to rrnB terminator and DNA replication origin region of pUC19 as a template. The base sequences at the 5'-ends of the primers (b-4) and (a-5) used to amplify this template DNA fragment are complementary to each other. In the overlap extension PCR, the combination of the primers (a-4) and (b-5) as described above was used to prepare the DNA for the multicloning region. PCR was performed according to a conventional method using "DNA thermal cycler" manufactured by Life Technologies Inc. and KOD FX Neo (manufactured by Toyobo Co., Ltd.) as a reaction reagent.

2 µL of the prepared reaction solution was electrophoresed on a 1% agarose gel, and a DNA fragment of about 1.0 kbp corresponding to the DNA in which rrnB terminator and the replication origin region of pUC19 were linked was detected.

### (4) Preparation of DNA fragment of neomycin/kanamycin resistance gene

The following pair of primers was synthesized and used to amplify the DNA fragment of neomycin/kanamycin resistance gene (hereinafter, referring to as "nptII gene") using pK18mobsacB [GenBank: FJ437239.1; Gene, 145, 69-73 (1994)] as a templatethe. PCR was performed according to a conventional method using "DNA thermal cycler" manufactured by Life Technologies Inc. and KOD FX Neo (manufactured by Toyobo Co., Ltd.) as a reaction reagent.

### Primers for the amplification of nptII gene

(a-6) 5'-TTGCTGGCCGCGGACGTAGAAAGCCAGTCCGCAGA-3' (SEQ ID NO: 41)
(b-6) 5'-GGCCCGGGTTATAAAAGCCAGTCATTAGGCCTATC-3' (SEQ ID NO: 42)

SmaI restriction site has been added to primer (b-6) as shown by the underline.

2 µL of the prepared reaction solution was electrophoresed on a 1% agarose gel, and a DNA fragment of about 1.0 kbp corresponding to nptII gene was detected.

### (5) Construction of circular plasmid in which DNA fragments prepared in (1) to (4) have been linked

In the DNA fragments prepared in the above (1) to (4), the end of the DNA fragment of (1) has the homologous sequence of 16 bp to the ends of the DNA fragments of (4) and (2), the end of the DNA fragment of (2) has the homologous sequence of 16 bp to the ends of the DNA fragments of (1) and (3), the end of the DNA fragment of (3) has the homologous sequence of 16 bp to the ends of the DNA fragments of (2) and (4), and the end of the DNA fragment of (4) has the homologous sequence of 16 bp to the ends of the DNA fragments of (3) and (1). Thus, the DNA fragments of (1), (2), (3), and (4) can be ligated to form a circular DNA using the Gibson Assembly. In order to ligate the DNA fragments prepared in (1) to (4) to form a circular DNA, the Gibson Assembly was performed using Gibson Assembly master mix manufactured by New England Biolabs. Escherichia coli JM109 was transformed with the prepared reaction solution by the calcium chloride method and plated to LB agar medium containing 50 µg/mL of kanamycin. The strain grown on the medium was liquid-cultured by a conventional method, the plasmid DNA was extracted from the culture medium, and this plasmid was cleaved with the restriction enzyme SmaI for confirmation. As a result, a DNA fragment of about 2.3 kbp, which corresponds to the size in which the DNA fragments prepared in (1) to (4) has been ligated to form a single DNA, was observed.

This circular plasmid DNA that replicates in E. coli was named pCAMO-1.

### (6) Construction of plasmid vector (pCAMO-4)

Hydrogenophilus thermoluteolus TH-1 strain (NBRC 14978) was inoculated into a test tube containing 5 mL of liquid medium A [(NH₄)₂SO₄ 3.0 g, KH₂PO₄ 1.0 g, K₂HPO₄ 2.0 g, NaCl 0.25 g, FeSO₄·7H₂O 0.014 g, MgSO₄·7H₂O 0.5 g, CaCl₂ 0.03 g, MoOs 4.0 mg, ZnSO₄·7H₂O 28 mg, CuSO₄·5H₂O 2.0 mg, H₃BO₃ 4.0 mg, MnSO₄·5H₂O 4.0 mg, and CoCl₂·6H₂O 4.0 mg were dissolved in 1 L of distilled water (pH 7.0)] using platinum loop, the tube was filled with a mixed gas of H₂ : O₂ : CO₂ = 7.5 : 1 : 1.5 and cultured with shaking at 50°C, and the culture solution was subjected to the conventional alkaline SDS method to obtain the endogenous plasmid pTH1 [Microbiol Resour Announc. 2018 Aug 16; 7 (6)].

The prepared pTH1 of about 66 kbp was cleaved with the restriction enzymes ScaI and PvuII, and the plasmid pCAMO-1 prepared above was cleaved with the restriction enzyme SmaI, and the cleaved plasmids were ligated each other using T4 DNA ligase (manufactured by Takara Bio Co., Ltd.).

Hydrogenophilus thermoluteolus TH-1 strain was transformed with the prepared ligation solution by an electric pulse method (electroporation method), and the solution was inoculated into solid medium A containing 50 µg/mL of kanamycin [(NH₄)₂SO₄ 3. 0 g, KH₂PO₄ 1.0 g, K₂HPO₄ 2.0 g, NaCl 0.25 g, FeSO₄·7H₂O 0.014 g, MgSO₄·7H₂O 0.5 g, CaCl₂ 0.03 g, MoOs 4.0 mg, ZnSO₄·7H₂O 28 mg, CuSO₄·5H₂O 2.0 mg, H₃BO₃ 4.0 mg, MnSO₄·5H₂O 4.0 mg, CoCl₂·6H₂O 4.0 mg, and 15 g of agar were dissolved in 1 L of distilled water (pH 7.0)], and the solution was cultured in a chamber filled with a mixed gas of H₂ : O₂ : CO₂ = 7.5 : 1 : 1.5 at 50°C for 60 hours. Seven strains grown on solid medium A were inoculated into a test tube containing 5 mL of liquid medium A containing 50 µg/mL of kanamycin using platinum loop, the test tube was filled with a mixed gas of H₂ : O₂ : CO₂ = 7.5 : 1 : 1.5 and cultured with shaking at 50°C, the plasmid DNA was extracted from the culture medium, and electrophoresis was performed with 1% agarose gel. As a result, it was confirmed that all 7 strains had the same plasmid DNA of about 5.5 kbp.

The following pair of primers corresponding to the both ends of SmaI restriction site in pCAMO-1 was synthesized and used to amplify the DNA fragment of the endogenous plasmid pTH1 inserted to SmaI restriction site of pCAMO-1 as a templatethe. PCR was performed according to a conventional method using "DNA thermal cycler" manufactured by Life Technologies Inc. and KOD FX Neo (manufactured by Toyobo Co., Ltd.) as a reaction reagent.

### Primers for the amplification of the DNA fragment of the inserted pTH1

(a-7) 5'-AATTGTCAGATAGGCCTAATGACTGGCTTTTATAA-3' (SEQ ID NO: 43)
(b-7) 5'-TGACGCCAGAAGCATTGGTGCACCGTGCAGTCGAT-3' (SEQ ID NO: 44)

2 µL of the prepared reaction solution was electrophoresed on a 1% agarose gel, and a DNA fragment of about 3.2 kbp was detected. In the obtained plasmid, a DNA fragment of about 3.2 kbp, which is a part of pTH1, has been inserted into the SmaI restriction enzyme site of the pCAMO-1 plasmid. Since this DNA fragment of about 3.2 kbp contains the replication origin region that works in the Hydrogenophilus thermoluteolus cell, the resulting plasmid replicates in the Hydrogenophilus thermoluteolus cell.

The constructed plasmid was named pCAMO-4 and used as a plasmid vector for introducing a gene into the Hydrogenophilus thermoluteolus.

### Transformants capable of producing aspartic acid and methionine

### Cloning of aspartate dehydrogenase gene

DNAs consisting of the base sequences of the aspartate dehydrogenase genes derived from bacteria selected as follows (Candidatus Hydrothermae bacterium (SEQ ID NO: 1), Glaciecola sp. 33A (SEQ ID NO: 2), Mesorhizobium sp. M7D.F.Ca.US.005.01.1.1 (SEQ ID NO: 3), Rhodospirillaceae bacterium (SEQ ID NO: 4), Candidatus Altiarchaeales archaeon IMC4 (SEQ ID NO: 13), Bacillus kochii (SEQ ID NO: 5), Novosphingobium rosa (SEQ ID NO: 6), Mumia flava (SEQ ID NO: 14), Archaeoglobus fulgidus (SEQ ID NO: 7), Arthrobacter sp. 161MFSha2.1 (SEQ ID NO: 8), Oceanotoga teriensis (SEQ ID NO: 9), Marinitoga sp. 1155 (SEQ ID NO: 10), Thermotogales bacterium (SEQ ID NO: 11), Thermotoga maritima (SEQ ID NO: 12), and Amycolatopsis thermoflava (SEQ ID NO: 15)) were entrusted to Eurofin Genomics Co., Ltd. for synthesis.

A blast search (database: NCBI) was performed using the amino acid sequence of the AspDH gene of Thermotoga maritima (SEQ ID NO: 27) as a query to select the sequences derived from 14 types of bacteria which were predicted as an AspDH gene. A blast search was performed on the amino acid sequences of AspDH reported in non-patent documents and the amino acid sequences (database: dgene) described in patent documents to determine the homology with the collected amino acid sequences. As a result, these amino acid sequences, except for Archaeoglobus fulgidus, showed very low homology of about 50%.

It was not clear if the polypeptides having the amino acid sequences derived from Candidatus Hydrothermae bacterium (SEQ ID NO: 16), Glaciecola sp. 33A (SEQ ID NO: 17), Mesorhizobium sp. M7D.F.Ca.US.005.01.1.1 (SEQ ID NO: 18), Rhodospirillaceae bacterium (SEQ ID NO: 19), Bacillus kochii (SEQ ID NO: 20), Novosphingobium rosa (SEQ ID NO: 21), Arthrobacter sp. 161MFSha2.1 (SEQ ID NO: 23), Oceanotoga teriensis (SEQ ID NO: 24), Marinitoga sp. 1155 (SEQ ID NO: 25), and Thermotogales bacterium (SEQ ID NO: 26) have AspDH activity.

### Construction of plasmid for the expression of AspDH gene

The plasmid in which the synthetic gene of AspDH has been cloned was cleaved with the restriction enzymes NdeI and NotI, and then electrophoresed on a 1% agarose gel. A DNA fragment corresponding to the AspDH synthesis gene was cut out from the agarose gel, and the gel was frozen and thawed to recover the DNA fragment of the AspDH synthesis gene.

The plasmid vector pCAMO-4 was cleaved with the restriction enzymes NdeI and NotI and ligated with the DNA fragment of the prepared AspDH synthesis gene using T4 DNA ligase (manufactured by Takara Bio Co., Ltd.). The endogenous plasmid was removed from the Hydrogenophilus thermoluteolus TH-1 strain by a conventional method to obtain a curing strain (TH-1C strain).

The Hydrogenophilus thermoluteolus TH-1C strain was transformed with the obtained ligation solution by the electric pulse method (electroporation method) and inoculated into solid medium A containing 50 µg/mL of kanamycin, and the solution was cultured in a chamber filled with a mixed gas of H₂ : O₂ : CO₂ = 7.5 : 1 : 1.5 at 50°C for 60 hours.

Each strain grown on solid medium A was inoculated into a test tube containing 5 mL of liquid medium A containing 50 µg/mL of kanamycin using platinum loop, and the test tube was filled with a mixed gas of H₂ : O₂ : CO₂ = 7.5 : 1 : 1.5 and cultured with shaking at 50°C, and the plasmid DNA was extracted from the culture medium. Each plasmid was cleaved with the restriction enzymes NdeI and NotI to confirm the inserted fragment. As a result, the DNA fragment of about 5.5 kbp of the plasmid pCAMO-4 and the inserted fragment of about 0.8 kbp in length corresponding to the AspDH gene were observed. The plasmids containing the AspDH genes derived from various bacteria and the Hydrogenophilus thermoluteolus TH-1C strains transformed with the plasmids were named as shown in Table 1 below.

### Production of aspartic acid and methionine

The aspartate dehydrogenase gene-introduced strain of Hydrogenophilus thermoluteolus prepared as described above was inoculated into liquid medium A containing 50 µg/mL of kanamycin using platinum loop, and the test tube was filled with a mixed gas of H₂ : O₂ : CO₂ = 7.5 : 1 : 1.5 and cultured with shaking at 50°C for 72 to 96 hours. After culturing, the cultured medium was centrifuged (4°C, 15,000 rpm, 1 minute) to quantify aspartic acid and methionine obtained in the medium supernatant. Aspartic acid and methionine were produced in the medium supernatant as shown in Table 1.

**[Table 1]**

| strain | plasmid | species from which gene is derived | Asp concentration in medium supernatant (µM) | Met concentration in medium supernatant (µM) |
|---|---|---|---|---|
| AspDH001 | pC-CanHyd-AspDH | Candidatus Hydrothermae bacterium | 10.9 | 4 |
| AspDH002 | pC-Gla33A-AspDH | Glaciecola sp. 33A | 37 | 0.9 |
| AspDH003 | pC-MesM7D-AspDH | Mesorhizobium sp. M7D.F.Ca.US.00 5.01.1.1 | 109.7 | 3.1 |
| AspDH004 | pC-RhoSpi-AspDH | Rhodospirillac eae bacterium | 46.4 | 9.4 |
| AspDH006 | pC-BacKoc-AspDH | Bacillus kochii | 60.5 | 3.8 |
| AspDH007 | pC-NovRos-AspDH | Novosphingobiu m rosa | 74.9 | 4.1 |
| AspDH009 | pC-ArcFul-AspDH | Archaeoglobus fulgidus | 0.9 | 5.3 |
| AspDH010 | pC-Art161-AspDH | Arthrobacter sp. 161MFSha2.1 | 137.8 | 1.1 |
| AspDH011 | pC-OceTer-AspDH | Oceanotoga teriensis | 55 | 9.7 |
| AspDH012 | pC-Mar1155-AspDH | Marinitoga sp. 1155 | 56.1 | 7.2 |
| AspDH013 | pC-The46-AspDH | Thermotogales bacterium 46 20 | 64.5 | 10.3 |
| AspDH014 | pC-TheMar-AspDH | Thermotoga maritima | 23.4 | 8.8 |
| AspDH005 (comparative example) | pC-CanAlt-AspDH | Candidatus Altiarchaeales archaeon IMC4 | 0.9 | 0.1 |
| AspDH008 (comparative example) | pC-MumFla-AspDH | Mumia flava | 0.7 | 0.1 |
| AspDH015 (comparative example) | pC-AmyThe-AspDH | Amycolatopsis thermoflava | 0 | 0.1 |
| pCAMO-4/TH-1C (comparative example) | pCAMO-4 | none | 2.9 | 1 |

As shown in Table 1, the Hydrogenophilus thermoluteolus strains of the present invention introduced aspartate dehydrogenase genes derived from various bacteria demonstrated that the concentration of aspartic acid was 0.9 to 137.8 µM and the concentration of methionine was 0.9 to 10.3 µM after culturing, which shows a significantly high ability to produce aspartic acid and methionine as compared with the control strain (the strains other than the bacteria of the present invention (AspDH005, 008, 015) and the strain not containing the AspDH gene (pCAMO-4/TH-1C)).

Also, all the strains described herein (including ATCC strain and NBRC strain) are internationally deposited under the Budapest Treaty or owned by institutions that can provide anyone without any conditions, commercially available, or can be prepared by a person skilled in the art according to the present specification and thus are publicly available.

### INDUSTRIAL APPLICABILITY

Since the transformant of the present invention can produce aspartic acid and/or methionine with high efficiency using carbon dioxide as a sole carbon source, it can produce chemical products with industrially high efficiency while solving global warming caused by an increase of carbon dioxide. That is, since the Hydrogenophilus bacterium used in the present invention has a particularly excellent carbon dioxide-fixing ability among organisms having a carbon dioxide-fixing ability, the transformant of the present invention can produce aspartic acid and/or methionine while fixing carbon dioxide on an industrial scale. Furthermore, since aspartic acid and/or methionine are raw materials for various chemicals and chemical products such as aspartame sweeteners, biodegradable polymers, feed additives and pharmaceuticals, the present invention can industrially produce products made from aspartic acid and/or methionine using only carbon dioxide as a carbon source without using saccharides with high cost and petroleum raw materials that cause a warming problem.

## Claims

1. A transformant produced by introducing an aspartate dehydrogenase gene into a Hydrogenophilus bacterium.

2. The transformant according to claim 1, wherein the aspartate dehydrogenase gene is a DNA selected from any of the followings (1) to (6):
(1) DNA, which consists of a base sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, or SEQ ID NO: 12;
(2) DNA, which consists of a base sequence having 90% or more identity to any one of SEQ ID NOs: 1 to 12, and encodes a polypeptide having aspartate dehydrogenase activity;
(3) DNA, which hybridizes with a DNA consisting of a base sequence complementary to any one of SEQ ID NOs: 1 to 12 under a stringent condition, and encodes polypeptide having an aspartate dehydrogenase activity;
(4) DNA, which encodes a polypeptide consisting of an amino acid sequence of SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, or SEQ ID NO 27;
(5) DNA, which encodes a polypeptide consisting of an amino acid sequence having 90% or more identity to any one of SEQ ID NOs: 16 to 27, and the polypeptide having aspartate dehydrogenase activity; and
(6) DNA, which encodes a polypeptide consisting of an amino acid sequence having a deletion, substitution, or addition of one or more amino acids in any one of SEQ ID NOs: 16 to 27, and the polypeptide having aspartate dehydrogenase activity.

3. The transformant according to claim 1 or 2, wherein the aspartate dehydrogenase gene is a DNA selected from any of the followings (1) to (6):
(1) DNA, which consists of a base sequence of SEQ ID NO: 3, SEQ ID NO: 8, SEQ ID NO: 9, or SEQ ID NO: 11;
(2) DNA, which consists of a base sequence having 90% or more identity to SEQ ID NO: 3, SEQ ID NO: 8, SEQ ID NO: 9, or SEQ ID NO: 11, and encodes a polypeptide having aspartate dehydrogenase activity;
(3) DNA, which hybridizes with a DNA consisting of a base sequence complementary to SEQ ID NO: 3, SEQ ID NO: 8, SEQ ID NO: 9, or SEQ ID NO: 11 under a stringent condition, and encodes a polypeptide having aspartate dehydrogenase activity;
(4) DNA, which encodes a polypeptide consisting of an amino acid sequence of SEQ ID NO: 18, SEQ ID NO: 23, SEQ ID NO: 24, or SEQ ID NO: 26;
(5) DNA, which encodes a polypeptide consisting of an amino acid sequence having 90% or more identity to SEQ ID NO: 18, SEQ ID NO: 23, SEQ ID NO: 24, or SEQ ID NO: 26, and the polypeptide having aspartate dehydrogenase activity; and
(6) DNA, which encodes a polypeptide consisting of an amino acid sequence having a deletion, substitution, or addition of one or more amino acids in an amino acid sequence of SEQ ID NO: 18, SEQ ID NO: 23, SEQ ID NO: 24, or SEQ ID NO: 26, and the polypeptide having aspartate dehydrogenase activity.

4. The transformant according to any one of claims 1 to 3, wherein the Hydrogenophilus bacterium is Hydrogenophilus thermoluteolus.

5. The transformant according to claim 4, wherein the Hydrogenophilus thermoluteolus is Hydrogenophilus thermoluteolus TH-1C strain.

6. A method for producing aspartic acid, which comprises a step of culturing the transformant according to any one of claims 1 to 5 using carbon dioxide as a substantially sole carbon source.

7. A method for producing methionine, which comprises a step of culturing the transformant according to any one of claims 1 to 5 using carbon dioxide as a substantially sole carbon source.
